# EUROPEAN PATENT SPECIFICATION

(11) **EP 0 787 494 B1**
(45) Date of publication and mention of the grant of the patent: **09.06.2004**
(21) Application number: 96928723.4
(22) Date of filing: 29.08.1996
(51) Int. Cl.: A61K 31/535, A61K 31/54, C07D 498/18, C07D 513/18, A61P 31/04

(54) **Use of rifamycin derivatives for the manufacture of a medicament for the treatment of diseases caused by infections of helicobacter pylori**
Verwendung eines Rifamycin-Derivates zur Herstellung eines Medikamentes zur Behandlung von Krankheiten verursacht durch Helicobacter pylori-Infektionen
Utilisation des derivates de rifamycine pour la manufacture d'un medicament contre les maladies causées par une infection par helicobacter pylori

(30) Priority: 01.09.1995 JP 22531795; 01.09.1995 JP 22531895
(43) Date of publication of application: 06.08.1997
(73) Proprietor: KANEKA CORPORATION, Osaka-shi, Osaka 530-8288 (JP)
(72) Inventor: YAMASHITA, Katsuji, Hyogo 654 (JP); YAMANE, Takehiko, Hyogo 655 (JP); SAKASHITA, Shinichi, Hyogo 657 (JP); HOSOE, Kazunori, Hyogo 676 (JP); FUJII, Kenji, Hyogo 674 (JP)
(74) Representative: VOSSIUS & PARTNER
(86) International application number: PCT/JP1996/002464
(87) International publication number: WO 1997/009047

(56) References cited:
- EP-A- 0 190 709
- EP-A- 0 366 914
- DE-A- 2 825 445
- GB-A- 1 081 757
- JP-A- 59 231 092
- US-A- 4 086 225
- K FUJII ET AL: "I VITRO AND IN VIVO ANTIBACTERIAL ACTIVITIES OF KRM-1648 AND KRM-1657, NEW RIFAMYCIN DERIVATIVES" , ANTIMICROBIAL AGENTS AND CHEMOTHERAPY,US,AMERICAN SOCIETY FOR MICROBIOLOGY, WASHINGTON, DC, VOL. 38, NR. 5, PAGE(S) 1118-1122 XP002085087 ISSN: 0066-4804 * abstract; figure 1 *
- YAMANE T ET AL: "SYNTHESIS AND BIOLOGICAL ACTIVITY OF 3'-HYDROXY-5'- AMINOBENZOXAZINORIFAMYCIN DERIVATIVES" , CHEMICAL AND PHARMACEUTICAL BULLETIN,JP,PHARMACEUTICAL SOCIETY OF JAPAN. TOKYO, VOL. 41, NR. 1, PAGE(S) 148-155 XP000197109 ISSN: 0009-2363 * compounds 15-26 * * abstract *
- DATABASE WPI Section Ch, Week 198506 Derwent Publications Ltd., London, GB; Class B02, AN 1985-035855 XP002130099 -& JP 59 231092 A (KANEBO LTD), 25 December 1984 (1984-12-25)
- CLIN. INFECT. DIS., 1996, Vol. 22, Suppl. 1, KUNIN, CAIVIN K., "Antimicrobial Activity of Rifabutin", S3-S14.

## Description

### TECHNICAL FIELD

The present invention relates to a medicine and a method for a medical treatment for diseases caused by the infection of Helicobacter pylori. More particularly, it relates to a curative medicine and a method for a medical treatment for maladies of digestive organs such as gastritis, gastroduodenitis, erosive gastritis, gastric erosion, erosive duodenitis, gastric ulcer, duodenal ulcer and so on, which are caused by the infection of Helicobacter pylori which is difficult to be removed by antibacterial agents such as usual antibiotic substances, synthetic antibacterial chemicals, and so on.

### BACKGROUND ART

Nowadays it is known that the infection of Helicobacter pylori onto a gastric epithelium of human being is a main factor for proceeding to either gastritis, gastric ulcer or duodenal ulcer and this is a possible factor for proceeding to stomach cancer. It is revealed that the relapse of gastric ulcer or duodenal ulcer is remarkably depressed by removal of Helicobacter pylori infecting to digestive, and various kinds of medicines, which are mainly antibacterial chemicals are tried to remove the bacteria. For instance, bismuth medicines examples of which are colloidal bismuth subcitrate, bismuth subsalicylate and so on, antibacterial chemical examples of which are amoxicillin, ampicillin, clarithromycin, ofloxacin, tetracycline and so on, antiprotozoal examples of which are tinidazole, metronidazole and so on, proton pump inhibitor examples of which are omeprazole, lansoprazole and so on, are tried to be administered alone or in combination with two or three kinds of them. However, for high removal effect of the bacteria, it is necessary to use a combination of the plural medicines, because it is not sufficient to use the medicine alone for that purpose. Further it is known that some strains of Helicobacter pylori separated from clinical samples have the resistance to usual medicines, and it is desired to develop a new medicine which is made effective for an improved removal of the bacteria and can be applied to the remedy for removing the bacteria of more patients.

Rifamycin derivatives like KRM-1648 or KRM-1657 have been shown to have antibacterial activity against bacterias like e.g. Staphylococcus aureus, Streptococcus pneumoniae, Enterococcus faecalis, Bacillus subtilis, Escherichia coli or Salmonella typhimurium (Fujii K. et al.: In vitro and in vivo antibacterial activities of KRM-1648 and KRM-1657, new rifamycin derivatives. Antimicrobial Agents and Chemotherapy. Vol. 38, no. 5 (1994), pages 1118-1122).

### DISCLOSURE OF THE INVENTION

As the result of an extensive study of inventors of this invention to develop new medicines for Helicobacter pylori, they have eventually found that rifamycin derivatives which are expressed by the following formula (I) have high antibacterial activity to Helicobacter pylori, and thus they have completed the present invention.

Specifically the present invention provides a curative medicine for a digestive organ disease caused by the infection of Helicobacter comprising as an effective component a rifamycin derivative expressed by the formula (I) or a physiologically acceptable salt thereof.

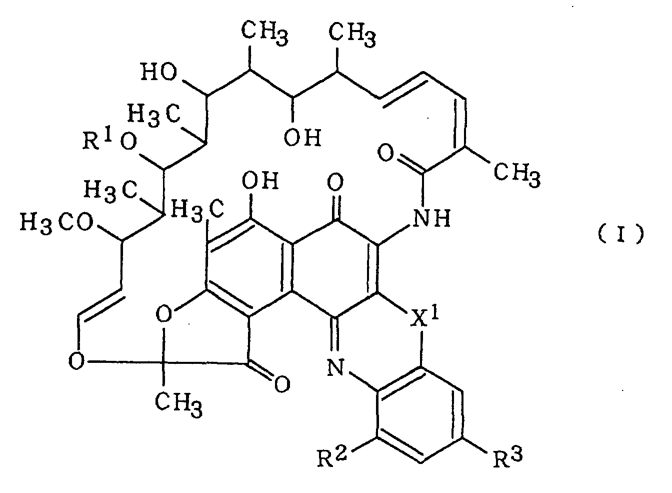

In the formula (I), X¹ represents an oxygen atom or a sulfur atom, R¹ represents an acetyl group or a hydrogen atom, R² represents a hydroxy group or a hydrogen atom, R³ represents 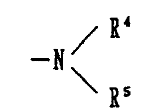 [in which R⁴ and R⁵ are the same or different and each is an alkyl group having 1 to 3 carbon atoms or a group expressed by the formula 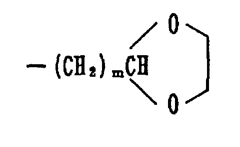 (in which m represents an integer between 1 and 3)], or 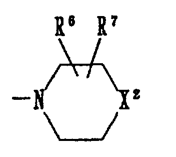 [in which R⁶ and R⁷ are the same or different and each is a hydrogen atom or an alkyl group having 1 to 3 carbon atoms, X² represents an oxygen atom or a sulfur atom or NR⁸ {R⁸ represents a hydrogen atom or an alkyl group having 1 to 6 carbon atoms or -(CH₂)ₙX³ (in which n represents an integer between 1 and 4, and X³ represents an alkoxy group having of 1 to 3 carbon atoms, or a vinyl group or an ethynyl group, or a group expressed by the formula

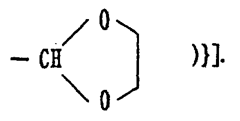

Further the present invention provides use of the rifamycin derivatives expressed by the formula (I) mentioned above or their physiologically acceptable salts for the production of a curative medicine for digestive organ diseases caused by the infection of Helicobacter.

More further the present invention provides a method for a medical treatment by the administration of the rifamycin derivatives expressed by the formula (I) mentioned above or their physiologically acceptable salts, for digestive organ diseases caused by the infection of Helicobacter.

In the formula (I) mentioned above, each of the alkyl groups expressed by R⁴, R⁵, R⁶ and R⁷, which consist of 1 to 3 members of carbon atoms, can be a methyl group, an ethyl group, a propyl group, an isopropyl group and a cyclopropyl group. The alkyl group expressed by R⁸, which consists of 1 to 6 members of carbon atoms, can be a chain or a cyclic alkyl group, examples of which may be a methyl group, an ethyl group, a propyl group, an isopropyl group, a cyclopropyl group, a butyl group, an isobutyl group, a sec-butyl group, a tert-butyl group, a cyclobutyl group, a cyclopropylmethyl group, a pentyl group, an isopentyl group, a sec-pentyl group, a tert-pentyl group, a cyclopentyl group, a cyclobutylmethyl group, a hexyl group, a 4-methylpentyl group, a cyclohexyl group, a 3-methylcyclopentyl group and so on.

The alkoxy group expressed by X³, which consists of 1 to 3 members of carbon atoms, can be a methoxy group, an ethoxy group, a propoxy group, an isopropoxy group and a cyclopropoxy group.

The rifamycin derivatives expressed by the formula (I), which are provided as the curative medicine for the disease caused by the infection of Helicobacter pylori, can be synthesized by the following method.

That is, they can be obtained by the method disclosed in Examined Japanese Patent (JP-B-3-58352), Examined Japanese Patent (JP-B-5-57275), Unexamined Japanese Patent (JP-A-3-7291), Unexamined Japanese Patent (JP-A-4-103589), Unexamined Japanese Patent (JP-A-3-101689), Chem. Pharm. Bull., 41, 148 (1993) and so on.

Among the rifamycin derivatives expressed by the formula (I), the derivatives, in which R¹ and R³ are the same as previously defined, R² represents a hydroxy group and X¹ represents a sulfur atom, can be synthesized by the following method. That is, the derivatives can be obtained by the method of reacting the compound expressed by the following formula (III) and the compound expressed by HR³ in an aprotic polar solvent such as N,N-dimethylformamide, N,N-dimethylacetamide, dimethyl sulfoxide and so on.

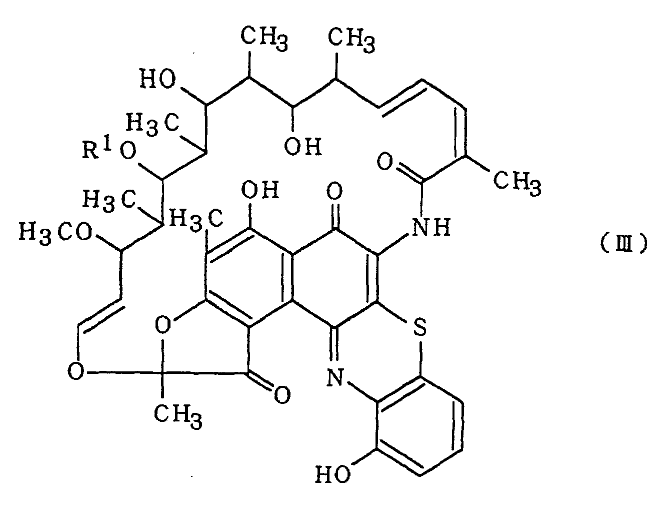

The rifamycin derivatives expressed by the formulas (I) can form salts with either acids or bases, and, as the acids or the bases which are used for forming the salts, any acid or base which can produce salts with the rifamycin derivatives can be selected arbitrarily. The concrete examples of the salts of bases are (1) metal salts, particularly salts of alkaline metals or alkali earth metals, (2) ammonium salts, (3) amine salts, particularly salts of methylamine or ethylamine or diethylamine or triethylamine or pyrrolidine or morpholine or hexamethyleneimine. On the other hand the concrete examples of the salts of acids are (1) salts of mineral acids, for example, sulfuric acid or hydrochloric acid, (2) salts of organic acids, for example, p-toluenesulfonic acid, trifluoroacetic acid or acetic acid.

The salts of the rifamycin derivatives in accordance with the present invention, which can be used as the medicines of the disease caused by the infection of Helicobacter pylori, can be available by selecting physiologically acceptable salts from the salts mentioned above.

Assays of antibacterial activity were carried out in order to examine the activity of the derivatives expressed by the formula (I) against Helicobacter pylori which is a pathogenic bacteria for digestive organs.

Assays of antibacterial activity of rifamycin derivatives expressed by the formula (I) were carried out by determination of minimal inhibitory concentration by agar plate dilution method using 5 strains (showed in Tables 1 and 2) and 10 strains (showed in Table 3) of Helicobacter pylori obtained by separation from clinical samples. As a culture medium, 5 % equine blood added blood agar culture medium No.2 (OXOID) was selected, and assayed compounds were added so that a constant concentration of it was obtained. After inoculation, the assayed bacteria was incubated at 35°C in 10 % cabon dioxide gas concentration, and the antibacterial activity was determined after 72 hours comparing with the results obtained by using the sample without assayed compound as a control. The results are shown in Tables 1 to 3. X¹, R¹, R² and R³ in Tables 1 to 3 correspond to those defined in the formula (I) mentioned above. In what follows, the derivatives correspond to those shown in Tables 1 to 3. MIC₈₀ is minimal inhibitory concentration (MIC) in which growth of 80 % of strains used in the assay are inhibited, and is shown in unit of µg/ml.

From the results in Tables 1 to 3, it is clearly seen that the rifamycin derivatives expressed by the formula (I), which are used as the curative medicine for the disease caused by the infection of Helicobacter pylori in accordance with the present invention, have extremely higher antibacterial activity compared with rifampicin which is a known rifamycin derivative used as antituberculosis agent.

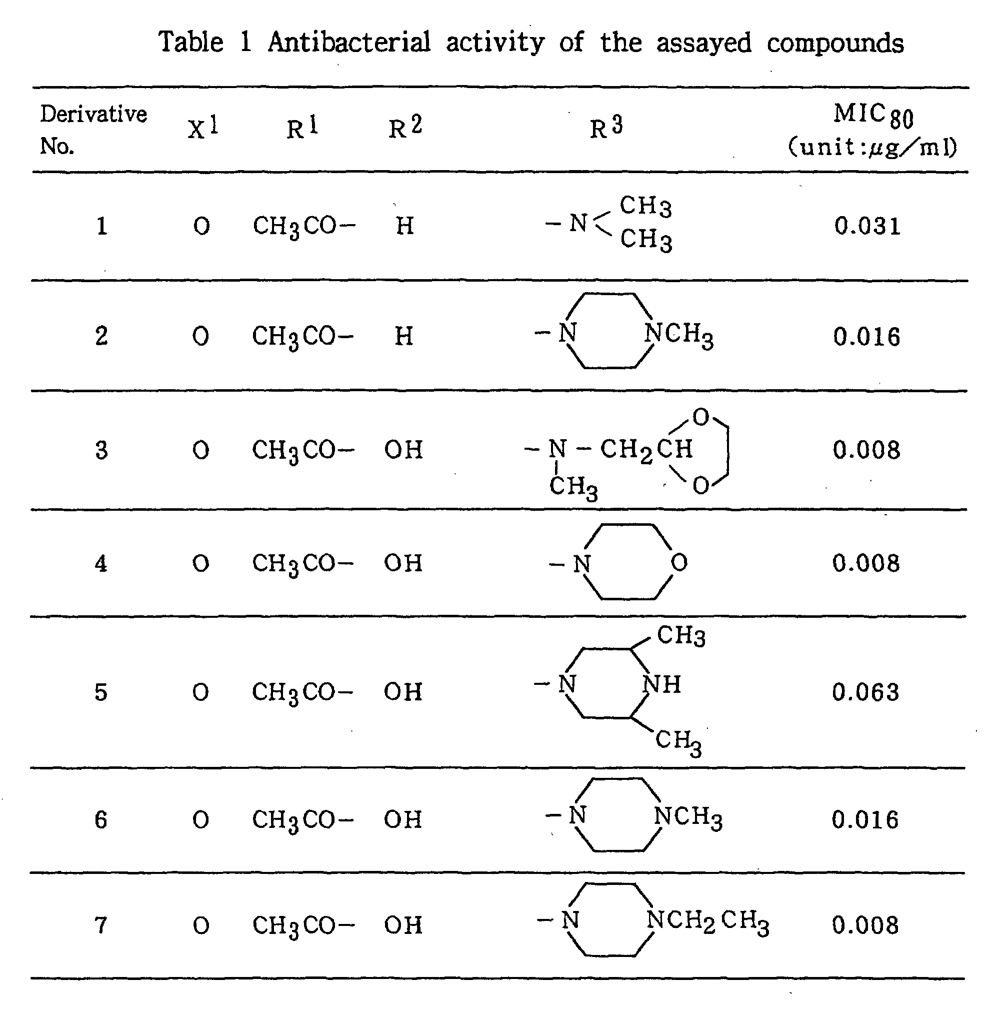

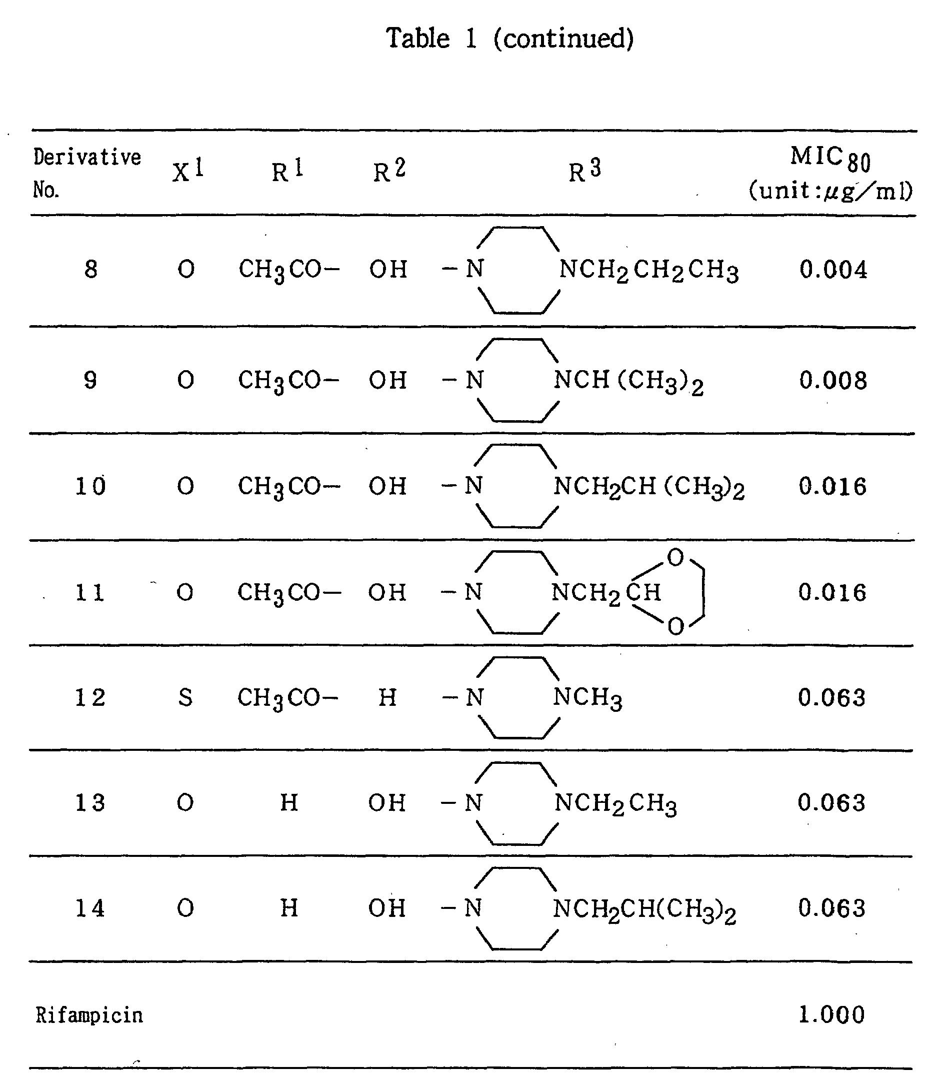

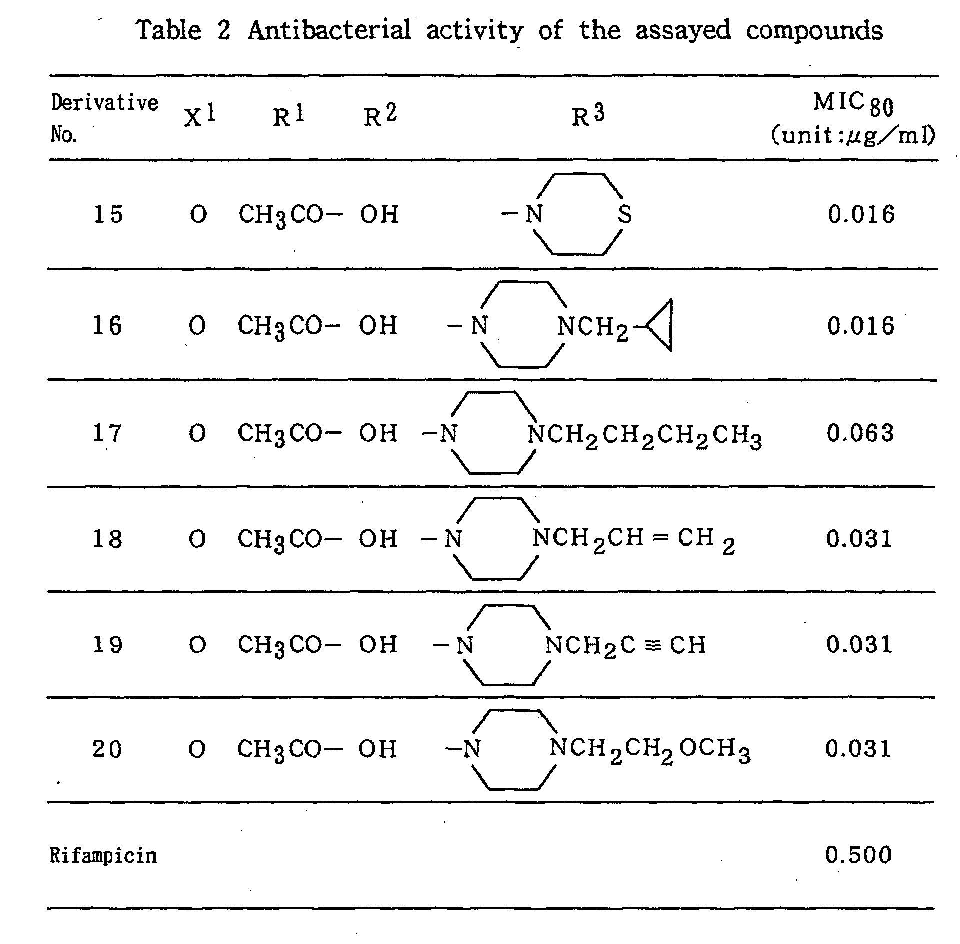

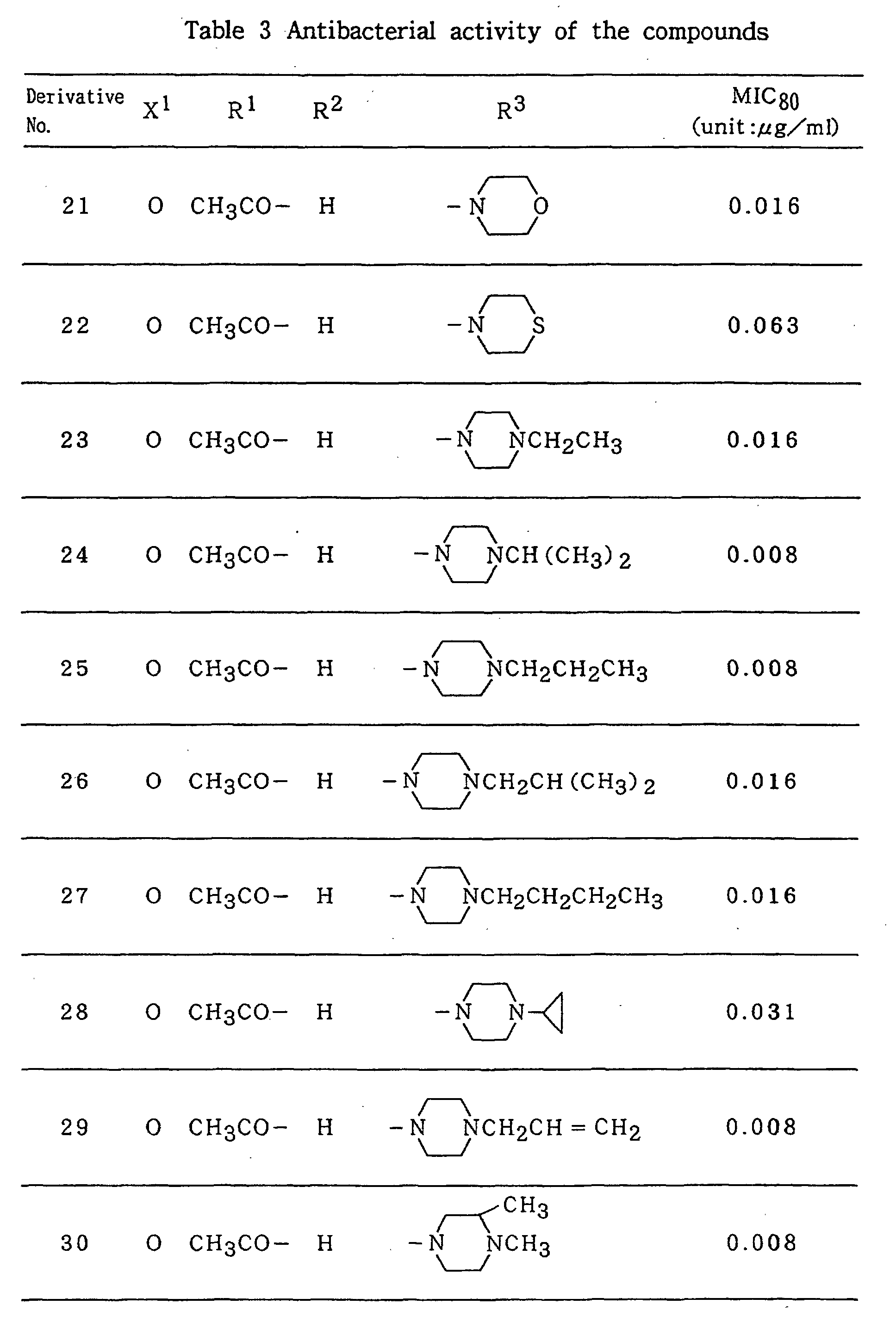

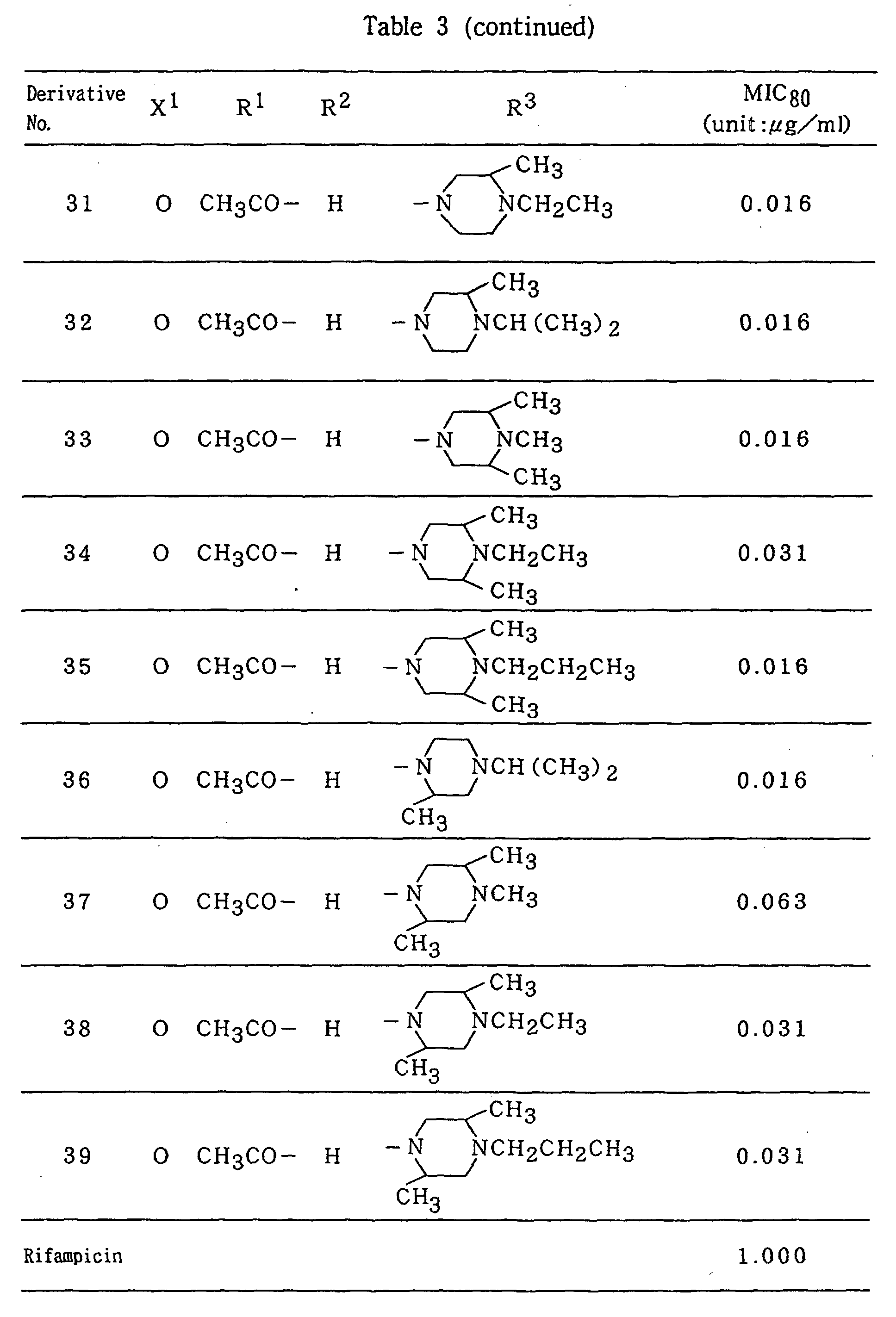

All rifamycin derivatives expressed by the formula (I), of which antibacterial activity is shown in Tables 1 to 3, have low toxicity, and the oral administration of each compound in a dose of 1,000 mg/kg to mice showed no toxicity.

The medicine of the present invention, the effective component of which is a rifamycin derivative expressed by the formula (I) or a physiologically acceptable salt thereof, is effective as a curative medicine for digestive organ diseases such as gastritis, gastroduodenitis, erosive gastritis, gastric erosion, erosive duodenitis, gastric ulcer, duodenal ulcer and so on, which are caused by the infection of Helicobacter pylori which is difficult to be removed by antibacterial agents such as usual antibiotic substances, synthetic antibacterial chemicals and so on.

The curative medicine for digestive organ disease caused by the infection of Helicobacter, the effective component of which is a rifamycin derivative expressed by the formula (I) or a physiologically acceptable salt thereof in the present invention, can be orally administered in the form of a powder, tablets, capsules, sugar-coated tablets, granules, syrup and so on. As the carrier for the preparation of the digestive organ disease curative medicine in the present invention, organic or inorganic solids or liquids suitable for oral administration, which are usually inert pharmaceutical carriers, can be used. Typical examples of the carrier are crystalline cellulose, gelatin, lactose, starch, magnesium stearate, talc, vegetable or animal fat or oil, gum, polyalkylene glycol and so on. The proportion of the effective component mentioned above in the medicine can be varied in the range of 0.2 to 100 % of the weight of the medicine. And the digestive organ disease curative medicine of the present invention can include other curative medicines for digestive organ disease and other medicines, which are compatible with it. Needless to say, in this case, the rifamycin derivative expressed by the formula (I) or its physiologically acceptable salt in the present invention may not be a main component in that medicine.

The curative medicine for the digestive organ disease in the present invention is usually administrated in such an amount that the desirable effect can be achieved without side-effects. Their actual dose should be determined by a doctor. Generally, however, the digestive organ disease curative medicine of the present invention is administered in a dose of 10 mg to 10 g, preferably 20 mg to 5 g, based on the amount of the effective component, per day for an adult. Further, the digestive organ disease curative medicine of the present invention can be adminstered in a unit dosage preparation containing 1 mg to 5 g, preferably 3 mg to 1 g of the effective component.

### BEST MODE FOR CARRYING OUT THE INVENTION

The present invention will be described in more detail with reference to the following Examples and Preparation Examples.

### EXAMPLE 1

A mixture of 100 g of derivative 8, 55 g of lactose and 41 g of dry potato starch was kneaded with 20 ml of water. The mixture was pressed out through a screen of 16 mesh, and dried at 40°C to be granulated. Then, the granules were uniformly mixed with 4 g of magnesium strearate and compressed into tablets by a conventional method to give tablets which contained 100 mg of derivative 8 in a 200 mg tablet.

### EXAMPLE 2

Using derivative 4 instead of derivative 8, tablets which contained 100 mg of derivative 4 in a 200 mg tablet were prepared by the same method as in Example 1.

### EXAMPLE 3

Using derivative 10 instead of derivative 8, tablets which contained 100 mg of derivative 10 in a 200 mg tablet were prepared by the same method as in Example 1.

### EXAMPLE 4

Using derivative 25 instead of derivative 8, tablets which contained 100 mg of derivative 25 in a 200 mg tablet were prepared by the same method as in Example 1.

### EXAMPLE 5

Using derivative 29 instead of derivative 8, tablets which contained 100 mg of derivative 29 in a 200 mg tablet were prepared by the same method as in Example 1.

### EXAMPLE 6

196 g of the granules which were prepared by the same method as in Example 1, were mixed with 4 g of magnesium stearate, and 200 mg portions of the resulting mixture was filled into No. 2 capsules to give hard capsules which contained 100 mg of derivative 8 in each capsule.

### EXAMPLE 7

Using derivative 4 instead of derivative 8 in Example 6, hard capsules which contained 100 mg of derivative 4 in each capsule were prepared by the same method as in Example 6.

### EXAMPLE 8

Using derivative 10 instead of derivative 8 in Example 6, hard capsules which contained 100 mg of derivative 10 in each capsule were prepared by the same method as in Example 7.

### EXAMPLE 9

10.0 g of derivative 3, 84.0 g of lactose, 4.5 g of crystalline cellulose and 1.5 g of magnesium stearate were mixed well to give a powder which contained 100 mg of derivative 3 in 1 g of the powder.

### EXAMPLE 10

Using derivative 6 instead of derivative 3 in Example 11, a powder which contained 100 mg of derivative 6 in 1 g of the powder was prepared by the same method as in Example 11.

### EXAMPLE 11

Using derivative 9 instead of derivative 3 in Example 11, a powder which contained 100 mg of derivative 9 in 1 g of the powder was prepared by the same method as in Example 11.

The method for producing the rifamycin derivatives in the present invention will be described with reference to the following Preparation Examples. In the following Preparation Examples, thin-layer chromatography was carried out using silica gel as a carrier, ¹H-NMR spectrum was measured in chloroform using tetramethyl silane as an internal standard and the position of the signals was expressed in ppm unit.

### PREPARATION EXAMPLE 1 (Synthesis of derivative 23)

1.57 g of benzoxazinorifamycin [synthesized by the method described in Helv. Chim. Acta 56, 2369 (1973)] was dissolved in 3.1 g of N,N-dimethylacetamide, and the mixture was heated up to 50°C.

To the mixture were added 0.46 g of N-ethylpiperazine and 0.52 g of manganese dioxide and the reaction was continued at 50°C for 14 hours. To dilute the reaction mixture, 30 ml of ethyl acetate was added. The solid in the reaction mixture was separated by filtration using diatomaceous earth as a filter aid and the residue on the funnel was rinsed with a small amount of ethyl acetate. The combined filtrate and rinse was washed with 30 ml of hydrochloric acid (0.03 mole/ℓ) once and with a saturated solution of sodium chloride twice, and dried over magnesium sulfate. The obtained ethyl acetate solution was evaporated under reduced pressure to remove the solvent. The resulting crude product was purified by silica gel column chromatography using 6 g of Wako Gel C-200 (commercial name of silica gel for column chromatography made by WAKO PURE CHEMICAL INDUSTRIES CO., LTD.) and chloroform as eluent. The fraction which contained the desired product was collected and evaporated to dryness under reduced pressure. The product was dissolved in 10 ml of ethyl acetate at 60°C and 20 ml of hexane was added to the solution. The solution was cooled slowly to room temperature to crystallize the product. The yield was 0.50 g.
Thin-layer chromatography:
Rf 0.26 blue spot (solvent : chloroform/methanol = 95/5 volume ratio), Rf 0.04 blue spot (solvent : toluene/tert-butanol = 9/1 volume ratio)
¹H-NMR:
The signals derived from the introduced N-ethylpiperazine:

   1.14(CH₂CH₃), 2.49(CH₂CH₃),

   2.60(NCH₂CH₂NCH₂CH₃),

   3.53(NCH₂CH₂NCH₂CH₃)

### PREPARATION EXAMPLE 2 (synthesis of derivative 24)

Using 0.51 g of N-isopropylpiperazine instead of N-ethylpiperazine in Preparation Example 1, the reaction was continued for 16 hours under the same condition as in Preparation Example 1. After the reaction mixture was treated in the same manner as in Preparation Example 1, the ethyl acetate solution was evaporated to dryness under reduced pressure to give a crude product. After the crude product was dissolved in 5 ml of ethyl acetate, 15 ml of hexane was added to the solution. The solution was cooled slowly to room temperature to crystallize the product. The yield was 0.44 g.
Thin-layer chromatography:
Rf 0.32 blue spot (solvent : chloroform/methanol = 95/5 volume ratio), Rf 0.10 blue spot (solvent : toluene/tert-butanol = 9/1 volume ratio)
¹H-NMR:
The signals derived from the introduced N-isopropylpiperazine:

   1.09(CH(CH₃)₂),

   2.67(NCH₂CH₂NCH(CH₃)₂),

   2.76(CH(CH₃)₂),

   3.52(NCH₂CH₂NCH(CH₃)₂)

### PREPARATION EXAMPLE 3 (synthesis of derivative 25)

Using 0.51 g of N-propylpiperazine instead of N-ethylpeperazine in Preparation Example 1, the reaction was continued for 15 hours under the same condition as in in Preparation Example 1. To the reaction mixture, 30 ml of ethyl acetate was added to dilute the mixture. The solid in the reaction mixture was separated by filtration using diatomaceous earth as a filter aid and the residue on the funnel was rinsed with a small amount of ethyl acetate. The combined filtrate and rinse was evaporated under reduced pressure. The product was dissolved in 10 ml of ethyl acetate, and 30 ml of hexane was added dropwise to the solution with agitating to precipitate the desired product. The obtained crude product was purified by silica gel column chromatography using 3 g of Wako Gel C-200 and chloroform as eluent. The fraction which contained the desired product was collected and evaporated to dryness under reduced pressure. After the crude product was dissolved in 10 ml of ethyl acetate at 60°C, 16 ml of hexane was added to the solution. The solution was cooled slowly to room temperature to crystallize the product. The yield was 0.69 g.
Thin-layer chromatography:
Rf 0.38 blue spot (solvent : chloroform/methanol = 95/5 volume ratio), Rf 0.13 blue spot (solvent : toluene/tert-butanol = 9/1 volume ratio)
¹H-NMR:
The signals derived from the introduced N-propylpiperazine:

   0.95(CH₂CH₂CH₃), 1.55(CH₂CH₂CH₃),

   2.37(CH₂CH₂CH₃), 2.59(NCH₂CH₂NCH₂CH₂CH₃),

   3.53(NCH₂CH₂NCH₂CH₂CH₃)

### PREPARATION EXAMPLE 4 (synthesis of derivative 26)

Using 0.57 g of N-isobutylpiperazine instead of N-ethylpeperazine in Preparation Example 1, the reaction was continued for 16 hours under the same condition as in Preparation Example 1. After the reaction mixture was treated in the same manner as in Preparation Example 1, the ethyl acetate solution was evaporated to dryness under reduced pressure to give a crude product. The obtained crude product was purified by silica gel column chromatography using 30 g of Wako Gel C-200 and toluene/tert-butanol = 95/5 (volume ratio) as eluent. The fraction which contained the desired product was collected and evaporated to dryness under reduced pressure. After the crude product was dissolved in 10 ml of ethyl acetate at 60°C, 30 ml of hexane was added to the solution. The solution was cooled slowly to room temperature to crystallize the product and the same process of crystallization was carried out once again. After the obtained crystalline product was dissolved in 10 ml of toluene at 60°C, 10 ml of hexane was added to the solution. The solution was cooled slowly to room temperature to crystallize the product. The obtained product was purified by silica gel column chromatography using 2 g of Wako Gel C-200 and toluene/tert-butanol = 95/5 (volume ratio) as eluent. After the partially purified product was dissolved in 5 ml of toluene at 60°C, 8 ml of hexane was added to the solution. The solution was cooled slowly to room temperature to crystallize the product. The obtained crystalline product was purified by preparative layer chromatography using silica gel 60 (E. Merck Inc.), 200×200×2 mm, and chloroform/methanol = 97/3 (volume ratio) as solvent. After the purified product was dissolved in 5 ml of toluene at 60°C, 15 ml of hexane was added to the solution. The solution was cooled slowly to room temperature to crystallize the product. The yield was 0.50 g.
Thin-layer chromatography:
Rf 0.54 blue spot (solvent : chloroform/methanol = 95/5 volume ratio), Rf 0.35 blue spot (solvent : toluene/tert-butanol = 9/1 volume ratio)
¹H-NMR:
The signals derived from the introduced N-isobutylpiperazine:

   0.93(CH₂CH(CH₃)₂),

   1.68(CH₂CH(CH₃)₂), 2.14(CH₂CH(CH₃)₂),

   2.55(NCH₂CH₂NCH₂CH(CH₃)₂),

   3.52(NCH₂CH₂NCH₂CH(CH₃)₂)

### PREPRATION EXAMPLE 5 (synthesis of derivative 27)

Using 0.57 g of N-butylpiperazine instead of N-ethylpiperazine in Preparation Example 1, the reaction was continued for 15 hours under the same condition as in Preparation Example 1. After the reaction mixture was treated in the same manner as in Preparation Example 1, the ethyl acetate solution was evaporated to dryness under reduced pressure to give a crude product. The obtained crude product was purified by silica gel column chromatography using 3 g of Wako Gel C-200 and chloroform as eluent. Further, silica gel column chromatography using 6 g of Wako Gel C-200 was carried out in the same way again. The fraction which contained the desired product was collected and evaporated to dryness under reduced pressure. After the product was dissolved in 10 ml of ethyl acetate at 60°C, 10 ml of hexane was added to the solution. The solution was cooled slowly to room temperature to crystallize the product. The yield was 0.74 g.
Thin-layer chromatography:
Rf 0.44 blue spot (solvent : chloroform/methanol = 95/5 volume ratio), Rf 0.19 blue spot (solvent : toluene/tert-butanol = 9/1 volume ratio)
¹H-NMR:
The signals derived from the introduced N-butyl piperazine:

   0.95(CH₂CH₂CH₂CH₃), 1.36 (CH₂CH₂CH₂CH₃),

   1.53(CH₂CH₂CH₂CH₃), 2.40(CH₂CH₂CH₂CH₃),

   2.59(NCH₂CH₂NCH₂CH₂CH₂CH₃),

   3.52(NCH₂CH₂NCH₂CH₂CH₂CH₃)

### PREPARATION EXAMPLE 6 (synthesis of derivative 28)

Using 0.50 g of N-cyclopropylpiperazine instead of N-ethylpiperazine in Preparation Example 1, the reaction was continued for 22 hours under the same condition as in Preparation Example 1. After the reaction mixture was treated in the same manner as in Preparation Example 1, the ethyl acetate solution was evaporated to dryness under reduced pressure to give a crude product. The obtained crude product was purified by silica gel column chromatography using 30 g of Wako Gel C-200 and toluene/tert-butanol = 95/5 (volume ratio) as eluent. The fraction which contained the desired product was collected and evaporated to dryness under reduced pressure. After the product was dissolved in 10 ml of toluene at 60°C, 10 ml of hexane was added to the solution. The solution was cooled slowly to room temperature to crystallize the product. The yield was 0.92 g.
Thin-layer chromatography:
Rf 0.49 blue spot (solvent : chloroform/methanol = 95/5 volume ratio), Rf 0.24 blue spot (solvent : toluene/tert-butanol = 9/1 volume ratio)
¹H-NMR:
The signals derived from the introduced N-cyclopropylpiperazine:

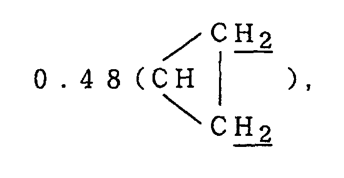

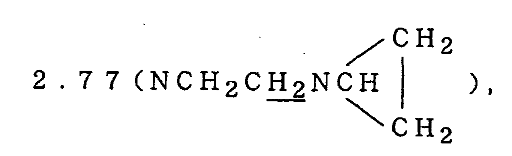

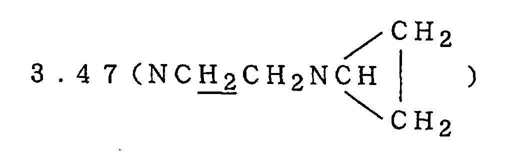

### PREPARTION EXAMPLE 7 (synthesis of derivative 29)

Using 0.50 g of N-(2-propenyl)piperazine instead of N-ethylpiperazine in Preparation Example 1, the reaction was continued for 15 hours under the same condition as in Preparation Example 1. After the reaction mixture was treated in the same manner as in Preparation Example 1, the ethyl acetate solution was evaporated to dryness under reduced pressure to give a crude product. The obtained crude product was purified by silica gel column chromatography using 6 g of Wako Gel C-200 and chloroform/methanol = 100/0 to 95/5 (volume ratio) as eluent. Further, silica gel column chromatography using 3 g of Wako Gel C-200 was again carried out using chloroform as eluent. The fraction which contained the desired product was collected and evaporated to dryness under reduced pressure. After the product was dissolved in 4 ml of toluene at 60°C, 10 ml of hexane was added to the solution. The solution was cooled slowly to room temperature to crystallize the product. The yield was 0.74 g.
Thin-layer chromatography:
Rf 0.40 blue spot (solvent : chloroform/methanol = 95/5 volume ratio), Rf 0.15 blue spot (solvent : toluene/tert-butanol = 9/1 volume ratio)
¹H-NMR:
The signals derived from the introduced N-(2-propenyl)-piperazine:

   2.61(NCH₂CH₂NCH₂CH=CH₂),

   3.07(CH₂CH=CH₂), 3.52(NCH₂CH₂NCH₂CH=CH₂),

   4.98,5.22(CH₂CH=CH₂), 5.88(CH₂CH=CH₂)

### PREPARATION EXAMPLE 8 (synthesis of derivative 30)

Using 0.46 g of 1,2-dimethylpiperazine instead of N-ethylpiperazine in Preparation Example 1, the reaction was continued for 16 hours under the same condition as in Preparation Example 1. After the reaction mixture was treated in the same manner as in Preparation Example 1, the ethyl acetate solution was evaporated to dryness under reduced pressure to give a crude product. The obtained crude product was twice purified by silica gel column chromatography using 3 g of Wako Gel C-200 and chloroform as eluent. The fraction which contained the desired product was collected and evaporated to dryness under reduced pressure. After the product was dissolved in 5 ml of toluene at 60°C, 10 ml of hexane was added to the solution. The solution was cooled slowly to room temperature to crystallize the product. The obtained crystalline product was purified by preparative layer chromatography using silica gel 60, 200×200×2 mm, and chloroform/methanol = 97/3 (volume ratio) as solvent. After the purified product was dissolved in 3 ml of toluene at 60°C, 10 ml of hexane was added to the solution. The solution was cooled slowly to room temperature to crystallize the product. The yield was 0.15 g.
Thin-layer chromatography:
Rf 0.22 blue spot (solvent : chloroform/methanol = 95/5 volume ratio), Rf 0.04 blue spot (solvent : toluene/tert-butanol = 9/1 volume ratio)
¹H-NMR:
The signals derived from the introduced 1,2-dimethylpiperazine:

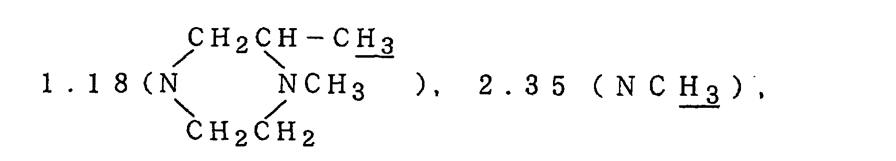

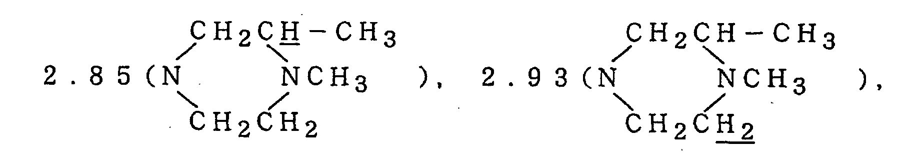

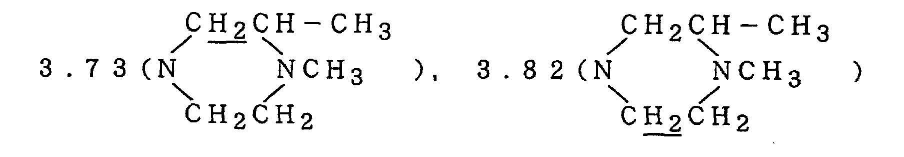

### PREPARTAION EXAMPLE 9 (synthesis of derivative 31)

Using 0.51 g of 1-ehtyl-2-methylpiperazine instead of N-ethylpiperazine in Preparation Example 1, the reaction was continued for 21 hours under the same condition as in Preparation Example 1. After the reaction mixture was treated in the same manner as in Preparation Example 1, the ethyl acetate solution was evaporated to dryness under reduced pressure to give a crude product. The obtained crude product was purified twice by silica gel column chromatography using 3 g of Wako Gel C-200 and chloroform as eluent. The fraction which contained the desired product was collected and evaporated to dryness under reduced pressure. After the product was dissolved in 10 ml of ethyl acetate at 60°C, 30 ml of hexane was added to the solution. The solution was cooled slowly to room temperature to crystallize the product. The yield was 0.51 g.
Thin-layer chromatography:
Rf 0.26 blue spot (solvent : chloroform/methanol = 95/5 volume ratio), Rf 0.06 blue spot (solvent : toluene/tert-butanol = 9/1 volume ratio)
¹H-NMR:
The signals derived from the introduced 1-ethyl-2-methylpiperazine:

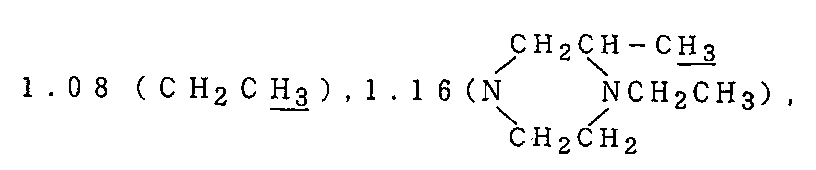

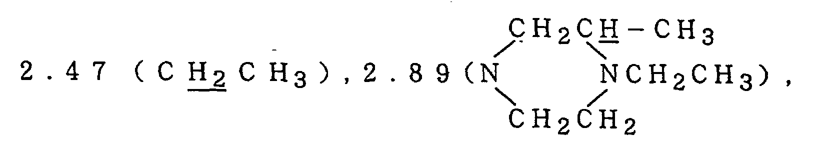

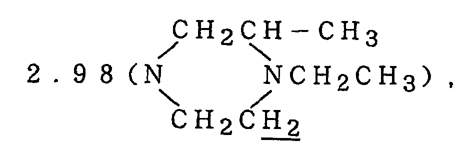

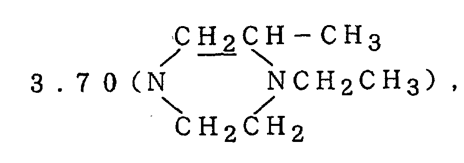

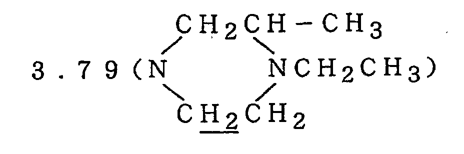

### PREPARTION EXAMPLE 10 (synthesis of derivative 32)

Using 0.57 g of 1-isopropyl-2-methylpiperazine instead of N-ethylpiperazine in Preparation Example 1, the reaction was continued for 20 hours under the same condition as in Preparation Example 1. After the reaction mixture was treated in the same manner as in Preparation Example 1, the ethyl acetate solution was evaporated to dryness under reduced pressure to give a crude product. The obtained crude product was twice purified by silica gel column chromatography using 3.5 g of Wako Gel C-200 and chloroform as eluent. Further the product was purified by silica gel column chromatography using 1.5 g of Wako Gel C-200 and chloroform as eluent. The fraction which contained the desired product was collected and evaporated to dryness under reduced pressure. After the product was dissolved in 5 ml of toluene at 60°C, 10 ml of hexane was added to the solution. The solution was cooled slowly to room temperature to crystallize the product. The yield was 0.37 g.
Thin-layer chromatography:
Rf 0.32 blue spot (solvent : chloroform/methanol = 95/5 volume ratio), Rf 0.13 blue spot (solvent : toluene/tert-butanol = 9/1 volume ratio)
¹H-NMR:
The signals derived from the introduced 1-isopropyl-2-methylpiperazine:

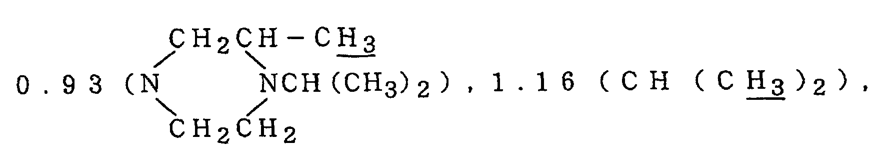

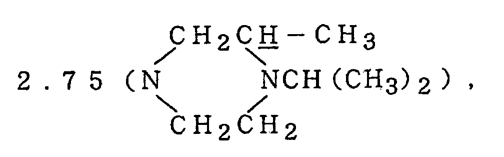

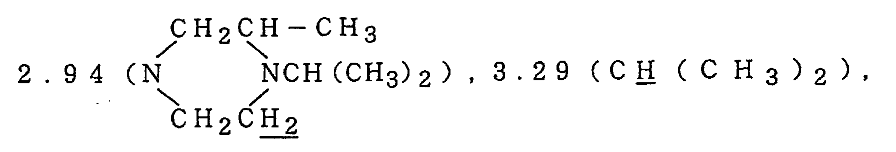

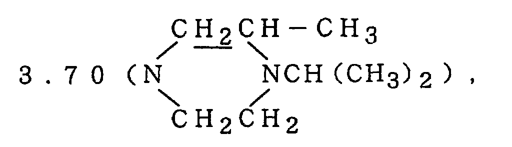

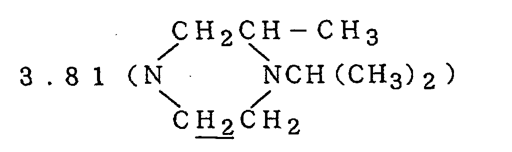

### PREPRATION EXAMPLES 11 (synthesis of derivative 33)

Using 0.51 g of 1,2,6-trimethylpiperazine instead of N-ethylpiperazine in Preparation Example 1, the reaction was continued for 16 hours under the same condition as in Preparation Example 1. After the reaction mixture was treated in the same manner as in Preparation Example 1, the ethyl acetate solution was evaporated to dryness under reduced pressure to give a crude product. The obtained crude product was purified by silica gel column chromatography using 30 g of Wako Gel C-200 and toluene/tert-butanol = 95/5 (volume ratio) as eluent. The fraction which contained the desired product was collected and evaporated to dryness under reduced pressure. After the product was dissolved in 10 ml of ethyl acetate at 60°C, 10 ml of hexane was added to the solution. The solution was cooled slowly to room temperature to crystallize the product. The yield was 0.44 g.
Thin-layer chromatography:
Rf 0.29 blue spot (solvent : chloroform/methanol = 95/5 volume ratio), Rf 0.07 blue spot (solvent : toluene/tert-butanol = 9/1 volume ratio)
¹H-NMR:
The signals derived from the introduced 1,2,6-trimethylpiperazine:

   1.22(NCH₂CH(CH₃)NCH₃),

   2.32(NCH₃), 2.88(NCH₂CH(CH₃)NCH₃),

   3.76(NCH₂CH(CH₃)NCH₃)

### PREPRATION EXAMPLE 12 (synthesis of derivative 34)

Using 0.57 g of 2,6-dimethyl-1-ethylpiperazine instead of N-ethylpiperazine in Preparation Example 1, the reaction was continued for 14 hours under the same condition as in Preparation Example 1. After the reaction mixture was treated in the same manner as in Preparation Example 1, the ethyl acetate solution was evaporated to dryness under reduced pressure to give a crude product. The obtained crude product was purified by silica gel column chromatography using 3 g of Wako Gel C-200 and chloroform as eluent. The fraction which contained the desired product was collected and evaporated to dryness under reduced pressure. After the product was dissolved in 5 ml of ethyl acetate at 60°C, 15 ml of hexane was added to the solution and the solution was cooled slowly to room temperature to crystallize the product and this crystallization process was conducted once more. Further the obtained crystalline product was purified by preparative layer chromatography using silica gel 60, 200×200×2 mm, and chloroform/methanol = 97/3 (volume ratio) as solvent. After the purified product was dissolved in 5 ml of toluene at 60°C, 15 ml of hexane was added to the solution. The solution was cooled slowly to room temperature to crystallize the product. The yield was 0.56 g.
Thin-layer chromatography:
Rf 0.33 blue spot (solvent : chloroform/methanol = 95/5 volume ratio), Rf 0.11 blue spot (solvent : toluene/tert-butanol=9/1 volume ratio)
¹H-NMR:
The signals derived from introduced 2,6-dimethyl-1-ethylpiperazine:

   0.93(CH₂CH₃),

   1.20(NCH₂CH(CH₃)NCH₂CH₃), 2.77(CH₂CH₃),

   2.98(NCH₂CH(CH₃)NCH₂CH₃), 3.76(NCH₂CH(CH₃)NCH₂CH₃)

### PREPRATION EXAMPLE 13 (synthesis of derivative 35)

Using 0.63 g of 2,6-dimethyl-1-propylpiperazine instead of N-ethylpiperazine in Preparation Example 1, the reaction was continued for 16 hours under the same condition as in Preparation Example 1. After the reaction mixture was treated in the same manner as in Preparation Example 1, the ethyl acetate solution was evaporated to dryness under reduced pressure to give a crude product. The obtained crude product was purified by silica gel column chromatography using 30 g of Wako Gel C-200 and chloroform/methanol = 95/5 (volume ratio) as eluent. And the obtained partially purified product was purified by silica gel column chromatography using 6 g of Wako Gel C-200 and chloroform as eluent, and further the same scale chromatography was done using toluene instead of chloroform as eluent. The obtained partially purified product was purified by preparative layer chromatography using silica gel 60, 200×200×2 mm, and chloroform/methanol = 95/5 (volume ratio) as solvent. The part which contained the desired product was scraped off and extracted with a solvent. The extract was evaporated to dryness. The yield was 0.23 g.
Thin-layer chromatography:
Rf 0.40 blue spot (solvent : chloroform/methanol = 95/5 volume ratio), Rf 0.18 blue spot (solvent : toluene/tert-butanol = 9/1 volume ratio)
¹H-NMR:
The signals derived from the introduced 2,6-dimethyl-1-propylpiperazine:

   0.86(CH₂CH₂CH₃),

   1.19(NCH₂CH(CH₃)NCH₂CH₂CH₃), 1.42(CH₂CH₂CH₃),

   2.75(CH₂CH₂CH₃), 2.75(NCH₂CH(CH₃)NCH₂CH₂CH₃),

   3.75(NCH₂CH(CH₃)NCH₂CH₂CH₃)

### PREPARATION EXAMPLE 14 (synthesis of derivative 36)

Using 0.57 g of 1-isopropyl-3-methylpiperazine instead of N-ethylpiperazine in Preparation Example 1, the reaction was continued for 119 hours under the same condition as in Preparation Example 1. After the reaction mixture was treated in the same manner as in Preparation Example 1, the ethyl acetate solution was evaporated to dryness under reduced pressure to give a crude product. The obtained crude product was purified by silica gel column chromatography using 6 g of Wako Gel C-200 and chloroform as eluent. Further the obtained partially purified product was purified three times by preparative layer chromatography using silica gel 60, 200×200×2 mm, and chloroform/methanol = 95/5 (volume ratio) as solvent. The part which contained the desired product was scraped off and extracted with a solvent. The extract was evaporated to dryness. The yield was 0.29 g.
Thin-layer chromatography:
Rf 0.47 blue spot (solvent : chloroform/methanol = 95/5 volume ratio), Rf 0.29 blue spot (solvent : toluene/tert-butanol = 9/1 volume ratio)
¹H-NMR:
The signals derived from the introduced 1-isopropyl-3-methylpiperazine:

   1. 06 ( C H (CH₃)₂,

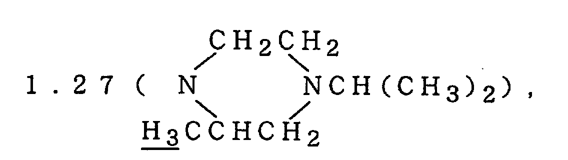

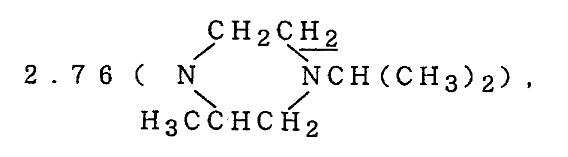

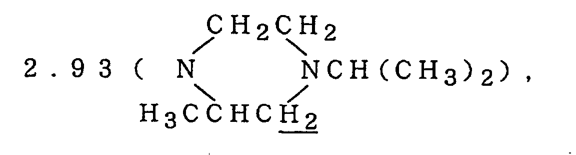

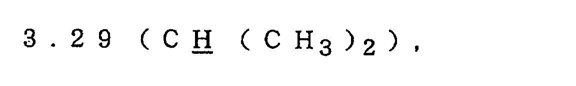

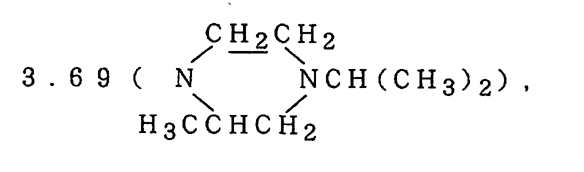

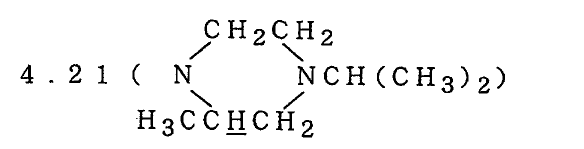

### PREPARTION EXAMPLE 15 (synthesis of derivative 37)

Using 0.51 g of 1,2,5-trimethylpiperazine instead of N-ethylpiperazine in Preparation Example 1, the reaction was continued for 45 hours under the same condition as in Preparation Example 1. After the reaction mixture was treated in the same manner as in Preparation Example 1, the ethyl acetate solution was evaporated to dryness under reduced pressure to give a crude product. The obtained crude product was purified by silica gel column chromatography using 6 g of Wako Gel C-200 and chloroform as eluent. Further the obtained partially purified product was purified by preparative layer chromatography using silica gel 60, 200×200×2 mm, and chloroform/methanol = 95/5 (volume ratio) as solvent. The obtained partially purified product was purified by preparative layer chromatography using silica gel 60, 200×200×2 mm, and chloroform/methanol = 95/5 (volume ratio) as solvent. Further the product was purified twice by the same preparative layer chromatography using chloroform/methanol = 90/10 (volume ratio) as solvent. The part which contained the desired product was scraped off and extracted with a solvent. The extract was evaporated to dryness. The yield was 0.24 g.
Thin-layer chromatography:
Rf 0.26 blue spot (solvent: chloroform/methanol = 95/5 volume ratio), Rf 0.08 blue spot (solvent : toluene/tert-butanol = 9/1 volume ratio)
¹H-NMR:
The signals derived from the introduced 1,2,5-trimethylpiperaz ine:

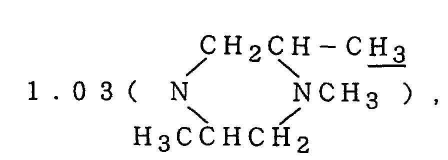

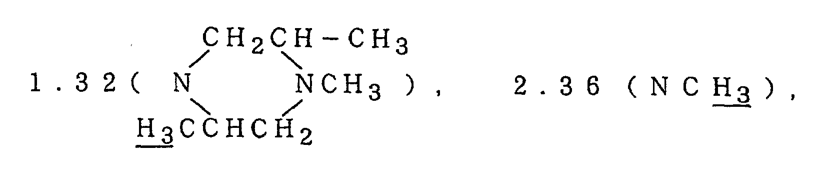

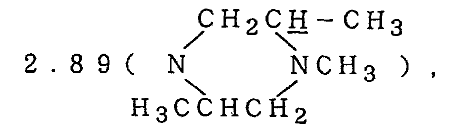

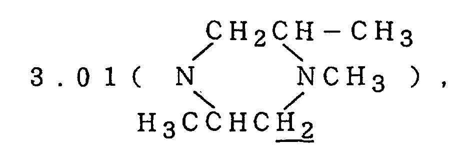

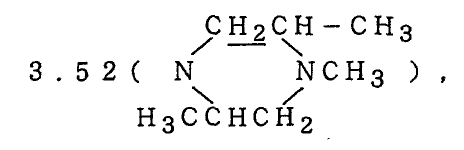

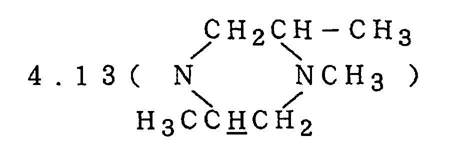

### PREPARATION EXAMPLE 16 (synthesis of derivative 38)

Using 0.57 g of 2,5-dimethyl-1-ethylpiperazine instead of N-ethylpiperazine in Preparation Example 1, the reaction was continued for 67 hours under the same condition as in Preparation Example 1. After the reaction mixture was treated in the same manner as in Preparation Example 1, the ethyl acetate solution was evaporated to dryness under reduced pressure to give a crude product. The obtained crude product was purified by silica gel column chromatography using 3 g of Wako Gel C-200 and toluene as eluent. The obtained partially purified product was purified twice by preparative layer chromatography using silica gel 60, 200×200×2 mm, and chloroform/methanol = 90/10 (volume ratio) as solvent. Further the product was purified by the same preparative layer chromatography using chloroform/methanol = 95/5 (volume ratio) as solvent. The obtained partially purified product was purified by silica gel column chromatography using 2 g of Wako Gel C-200 and toluene as eluent. The fraction which contained the desired product was collected and evaporated to dryness. The yield was 0.12 g.
Thin-layer chromatography:
Rf 0.37 blue spot (solvent : chloroform/methanol = 95/5 volume ratio), Rf 0.15 blue spot (solvent : toluene/tert-butanol = 9/1 volume ratio)
¹H-NMR:
The signals derived from the introduced 2,5-dimethyl-1-ethylpiperazine:

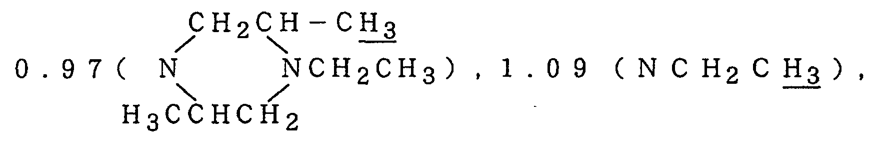

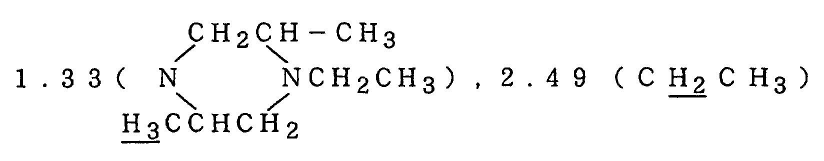

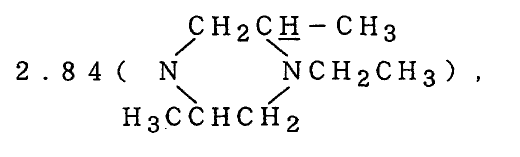

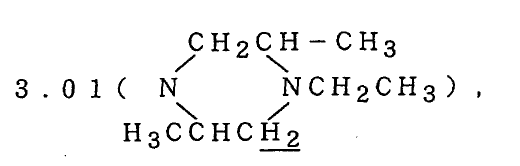

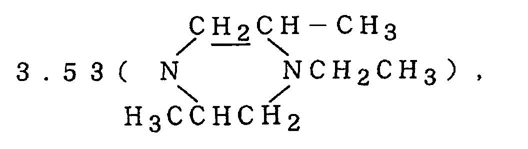

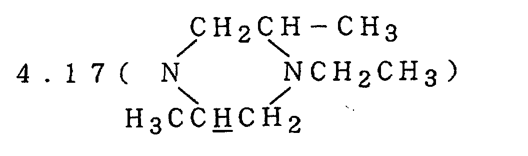

### PREPARTION EXAMPLE 17 (synthesis of derivative 39)

Using 0.63 g of 2,5-dimethyl-1-propylpiperazine instead of N-ethylpiperazine in Preparation Example 1, the reaction was continued for 119 hours under the same condition as in Preparation Example 1. After the reaction mixture was treated in the same manner as in Preparation Example 1, the ethyl acetate solution was evaporated to dryness under reduced pressure to give a crude product. The obtained crude product was purified by silica gel column chromatography using 10 g of Wako Gel C-200 and chloroform as eluent. The obtained partially purified product was purified by silica gel column chromatography using 3 g of Wako Gel C-200 and chloroform as eluent. The obtained partially purified product was purified by preparative layer chromatography using silica gel 60, 200X200X2 mm, and chloroform/methanol = 95/5 (volume ratio) as solvent. Further the obtained product was purified by the same preparative layer chromatography using chloroform/methanol = 98/2 (volume ratio) as solvent. The part which contained the desired product was scraped off and extracted with a solvent. The extract was evaporated to dryness. The yield was 0.21 g.
Thin-layer chromatography:
Rf 0.51 blue spot (solvent : chloroform/methanol = 95/5 volume ratio), Rf 0.31 blue spot (solvent : toluene/tert-butanol = 9/1 volume ratio)
¹H-NMR:
The signals derived from the introduced 2,5-dimethyl-1-propyl piperazine:

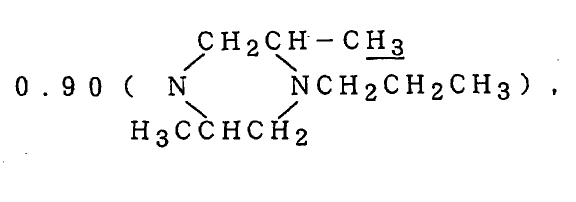

   0. 95 ( C H₂ C H₂ C H₃ ) ,

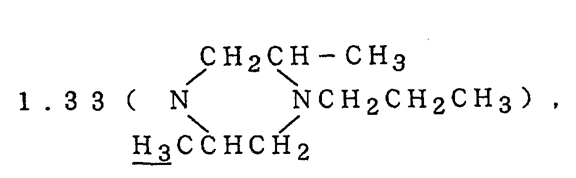

   1. 49 ( C H₂ C H₂ C H₃ ) , 2 . 3 9 ( C H₂ C H₂ C H₃ ) ,

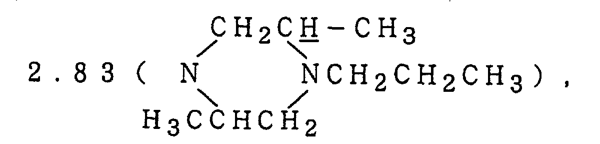

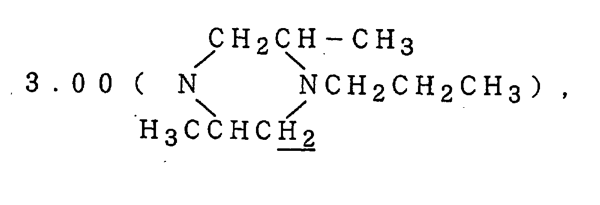

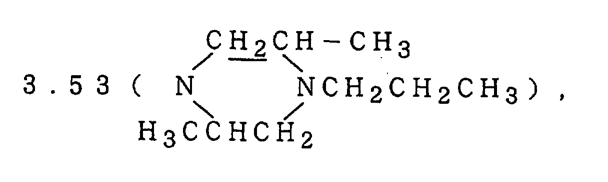

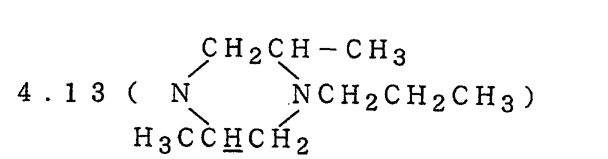

The present invention provides the new medicine, the effective ingredient of which is rifamycin derivatives or their physiologically acceptable salts, for diseases caused by the infection of Helicobacter pylori.

## Claims

1. Use of a rifamycin derivative expressed by the formula (I), or a physiologically acceptable salt thereof for the production of a curative medicine for a digestive organ disease caused by the infection of Helicobacter pylori 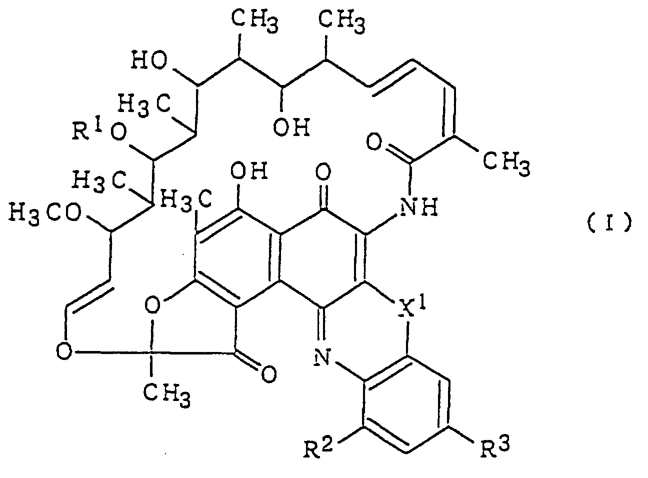 wherein , X¹ represents an oxygen atom or a sulfur atom, R¹ represents an acetyl group or a hydrogen atom, R² represents a hydroxy group or a hydrogen atom, R³ represents 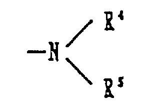 [in which R⁴ and R⁵ are the same or different and each is an alkyl group having 1 to 3 carbon atoms or a group expressed by the formula 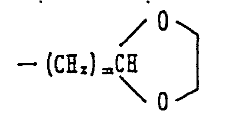 (in which m represents an integer between 1 and 3)], or 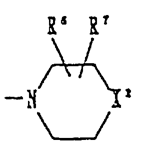 [in which R⁶ and R⁷ are the same or different and each is a hydrogen atom or an alkyl group having 1 to 3 carbon atoms, X² represents an oxygen atom or a sulfur atom or NR⁸ (R⁸ represents a hydrogen atom or an alkyl group having 1 to 6 carbon atoms or -(CH₂)ₙX³ (in which n represents an integer between 1 and 4, and X³ represents an alkoxy group having of 1 to 3 carbon atoms, or a vinyl group or an ethynyl group, or a group expressed by the formula

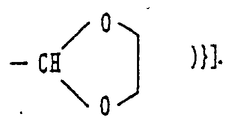

## Patentansprüche

1. Verwendung eines Rifamycin-Derivates der Formel (I) oder eines physiologisch verträglichen Salzes davon zur Herstellung eines heilenden Medikaments für eine Erkrankung des Verdauungsorgans, hervorgerufen durch eine Infektion mit Helicobacter pylori 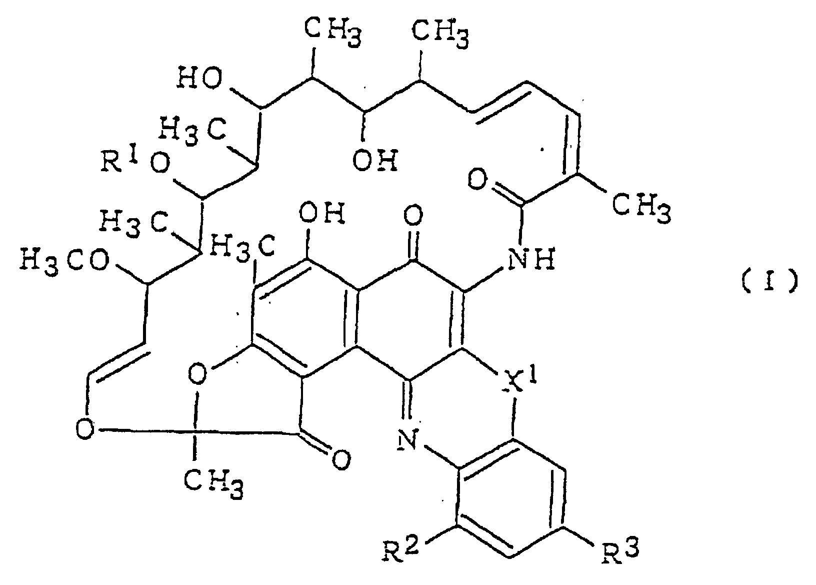 wobei X¹ ein Sauerstoffatom oder ein Schwefelatom darstellt, R¹ einen Acetylrest oder ein Wasserstoffatom darstellt, R² eine Hydroxygruppe oder ein Wasserstoffatom darstellt, R³ den Rest 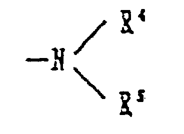 [wobei R⁴ und R⁵ gleich oder verschieden sind und jeweils einen Alkylrest mit 1 bis 3 Kohlenstoffatomen oder einen Rest der Formel 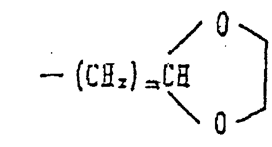 darstellen (wobei m eine ganze Zahl zwischen 1 und 3 ist)], oder den Rest 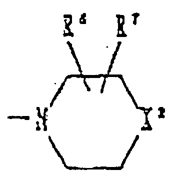 darstellt [wobei R⁶ und R⁷ gleich oder verschieden sind und jeweils ein Wasserstoffatom oder einen Alkylrest mit 1 bis 3 Kohlenstoffatomen darstellen, X² ein Sauerstoffatom oder ein Schwefelatom oder NR⁸ darstellt {R⁸ stellt ein Wasserstoffatom oder einen Alkylrest mit 1 bis 6 Kohlenstoffatomen oder -(CH₂)ₙX³ dar (wobei n eine ganze Zahl zwischen 1 und 4 ist, und X³ einen Alkoxyrest mit 1 bis 3 Kohlenstoffatomen darstellt, oder eine Vinylgruppe oder eine Ethinylgruppe, oder eine Gruppe der Formel 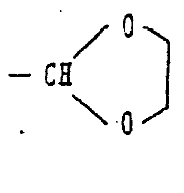 darstellt)}].

## Revendications

1. Utilisation d'un dérivé de rifamycine exprimé par la formule (I), ou d'un sel physiologiquement acceptable de celui-ci pour la production d'un médicament curatif pour une maladie de l'appareil digestif causée par l'infection à *Helicobacter pylori* : 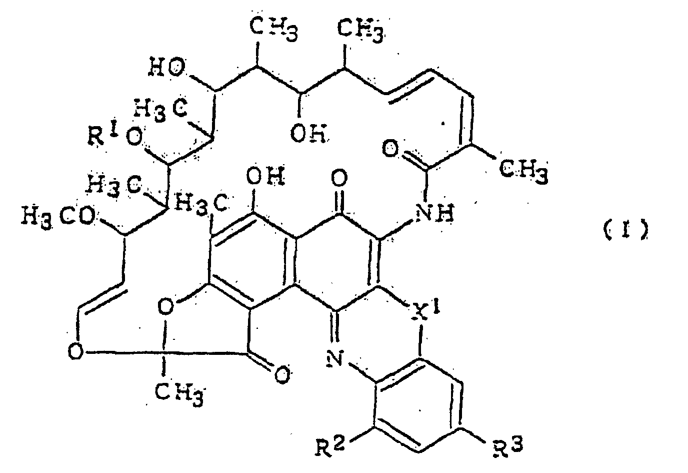 dans laquelle, X¹ représente un atome d'oxygène ou un atome de soufre, R¹ représente un groupe acétyle ou un atome d'hydrogène, R² représente un groupe hydroxy ou un atome d'hydrogène, R³ représente 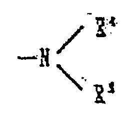 [dans lequel R⁴ et R⁵ sont identiques ou différents et chacun est un groupe alkyle ayant 1 à 3 atomes de carbone ou un groupe exprimé par la formule 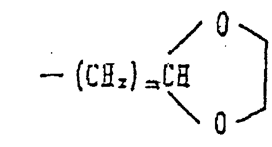 (dans lequel m représente un entier entre 1 et 3)], ou 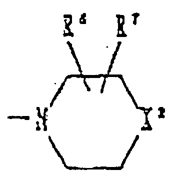 [dans lequel R⁶ et R⁷ sont identiques ou différents et chacun est un atome d'hydrogène ou un groupe alkyle ayant 1 à 3 atomes de carbone, X² représente un atome d'oxygène ou un atome de soufre ou NR⁸ {R⁸ représente un atome d'hydrogène ou un groupe alkyle ayant 1 à 6 atomes de carbone ou -(CH₂)ₙX³ (dans lequel n représente un entier entre 1 et 4, et X³ représente un groupe alcoxy ayant 1 à 3 atomes de carbone, ou un groupe vinyle ou un groupe éthynyle, ou un groupe exprimé par la formule
